(19) Europäisches Patentamt
European Patent Office
Office européen des brevets

(11) Veröffentlichungsnummer: **0 207 331**
**B1**

(12) **EUROPÄISCHE PATENTSCHRIFT**

(45) Veröffentlichungstag der Patentschrift: **23.05.90**

(21) Anmeldenummer: **86107890.5**

(22) Anmeldetag: **10.06.86**

(51) Int. Cl.$^5$: **C 07 D 295/12,**
A 61 K 31/445, A 61 K 9/00

# (54) Neue feste Formen von 2-Äthoxy-4-N-(1-(2-piperidino-phenyl)-3-methyl-1-butyl)-aminocarbonylmethylr-benzoesäure, diese Formen enthaltende Arzneimittel und Verfahren zu ihrer Herstellung.

(30) Priorität: **25.06.85 DE 3522604**

(43) Veröffentlichungstag der Anmeldung:
**07.01.87 Patentblatt 87/02**

(45) Bekanntmachung des Hinweises auf die Patenterteilung:
**23.05.90 Patentblatt 90/21**

(84) Benannte Vertragsstaaten:
**AT BE CH DE FR GB IT LI LU NL SE**

(56) Entgegenhaltungen:
**EP-A-0 147 850**

(73) Patentinhaber: **Dr. Karl Thomae GmbH**
**Postfach 1755**
**D-7950 Biberach (Riss) (DE)**

(72) Erfinder: **Grell, Wolfgang, Dr. Dipl.-Chem.**
**Amriswilstrasse 7**
**D-7950 Biberach 1 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 207 331 B1

**Beschreibung**

In der EP—A—0.099.017 werden bereits 4-[N-(1-(2-Piperidinophenyl)-1-alkyl)-aminocarbonylmethyl]benzoesäuren beschrieben, in denen die Alkylgruppe eine n-Butylgruppe oder eine Alkylgruppe mit 5 bis 8 Kohlenstoffatome darstellen kann, welche eine Wirkung auf den Stoffwechsel, insbesondere jedoch eine blutzuckersenkende Wirkung, aufweisen.

Außerdem wird in der nicht vorveröffentlichten EP—A—0.147.850 die Verbindung 2-Äthoxy-4-[N-(1-(2-piperidino-phenyl)-3-methyl-1-butyl)-aminocarbonylmethyl]-benzoesäure mit einem Schmelzpunkt von 90—92°C (Aceton/Petroläther; Form (A)) beschrieben. Diese Verbindung wird hierbei gemäß Beispiel 11 durch katalytische Hydrierung von 2-Äthoxy-4-[N-(1-(2-piperidino-phenyl)-3-methyl-1-buten-1-yl)-aminocarbonylmethyl]-benzoesäure erhalten; sie weist, wie ihre physiologisch verträglichen Additionssalze und ihre Enantiomeren wertvolle pharmakologische Eigenschaften auf, nämlich eine Wirkung auf den Intermediärstoffwechsel, insbesondere eine blutzuckersenkende Wirkung.

Überraschenderweise wurde nun gefunden, daß man die gleiche Verbindung, nämlich 2-Äthoxy-4-[N-(1-(2-piperidino-phenyl)-3-methyl-1-butyl)-aminocarbonylmethyl]-benzoesäure, beim Kristallisieren aus anderen Lösungsmitteln oder Lösungsmittelgemischen in zwei weiteren festen Formen erhält, welche als Form (B) und als Form (C) bezeichnet werden.

Gegenstand der vorliegenden Erfindung sind somit die neuen Formen (B) und (C) der 2-Äthoxy-4-[N-(1-(2-piperidino-phenyl)-3-methyl-1-butyl)-aminocarbonylmethyl]-benzoesäure, Arzneimittel, welche diese Formen enthalten, Verfahren zur Herstellung der neuen Formen und zur Herstellung von Arzneimitteln, welche diese Formen enthalten.

Die Form (B), welche einen Schmelzpunkt von 140 bis 142°C aufweist, erhält man durch Kristallisation aus Äthanol/Wasser (2/1); die schaumartige Form (C), die einen Schmelzbereich von 75 bis 85°C aufweist, erhält man aus dem 1:1-Methanol-Addukt (Schmelzpunkt: 85 bis 90°C), das bei Kristallisation aus Methanol anfällt, durch Erwärmen bei 60°C im Vakuum (5 Torr) über Phosphorpentoxid, wobei das Methanol entfernt wird.

Die Formen sind im gelösten Zustand identisch, wie aus den entsprechenden Lösungs-Spektren, z.B. den IR-Spektren in Methylenchlorid (siehe Abbildungen A, B und C) eindeutig hervorgeht. Dagegen unterscheiden sich die Formen im festen Zustand durch ihr Schmelzverhalten und durch ihre Feststoff-Spektren, z.B. durch die entsprechenden IR-KBr-Spektren (siehe Abbildungen A′, B′ und C′).

Zur Messung der Infrarot-Absorption wurden die Formen (A), (B) und (C) in Methylenchlorid gelöst (40 mg Substanz/ml Methylenchlorid) bzw. mit Kaliumbromid innig verrieben und dann hydraulisch zu einer Tablette verpreßt (ca. 1 mg Substanz/300 mg KBr).

Die IR-Spektren wurden im Falle der Lösungen mit einem IR-Spektrometer (Perkin-Elmer Typ 299) in einer Natriumchlorid-Küvette (Schichtdicke 0,2 mm) im Vergleich zu einer reinen Methylenchlorid-Lösung und im Falle der Kaliumbromid-Preßlinge mit einem IR-Spektrometer (Perkin-Elmer Typ 298) im Vergleich zu Luft gemessen.

Die Formen lassen sich durch entsprechende Umkristallisation und Trocknung ineinander überführen. So erhält man durch Umkristallisation der hochschmelzenden Form (B) aus Aceton/Petroläther die niedrigschmelzende Form (A), und durch Umkristallisation der niedrigschmelzenden Form (A) aus Ethanol/Wasser erhält man die hochschmelzende Form (B). Durch Umkristallisation der hochschmelzenden Form (B) aus Methanol erhält man ein 1:1-Addukt mit Methanol und aus diesem durch Entfernen des Methanols die schaumartige Form (C).

Unabhängig von der Art des angewendeten Verfahrens für die Synthese der erfindungsgemäßen Verbindung kann man also durch die Wahl des Lösungsmittels oder des Lösungsmittelgemisches beim Kristallisieren sowie durch entsprechende Trocknung gewünschtermaßen die hochschmelzende oder die niedrigschmelzende Form oder die schaumartige Form herstellen. Dies ist von Bedeutung für die praktische Verwendung der festen Formen, sei es ohne oder mit galenischen Hilfstoffen in Arzneimitteln, insbesondere zur Senkung des Blutzuckers bei der Behandlung des Typ-II-Diabetes; denn unterschiedliche feste Formen können unterschiedliche Haltbarkeit aufweisen und/oder in vivo unterschiedliches Resorptionsverhalten und somit unterschiedlichen Verlauf der biologischen Wirkung zur Folge haben.

Erfindungsgemäß erhält man die Verbindung 2-Äthoxy-4-[N-(1-(2-piperidino-phenyl)-3-methyl-1-butyl)-aminocarbonylmethyl]benzoesäure nach den in der vorstehend erwähnten nicht vorveröffentlichten Europäischen Offenlegungsschrift beschriebenen Verfahren, vorzugsweise jedoch durch Umsetzung von 3-Methyl-1-(2-piperidino-phenyl)-1-butylamin mit einer Verbindung der allgemeinen Formel

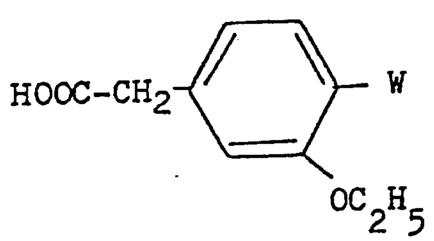

in der

W eine Carboxygruppe oder eine durch einen Schutzrest geschützte Carboxylgruppe darstellt, oder mit deren gegebenenfalls im Reaktionsgemisch hergestellten reaktionsfähigen Derivaten, erforderlichenfalls anschließende Abspaltung eines verwendeten Schutzrestes und die neuen festen Formen (B) und (C) durch anschließende entsprechende Kristallisation, durch abschließende entsprechende Umkristallisation und/oder entsprechende Trocknung.

Als reaktionsfähige Derivate einer Verbindung der vorstehend erwähnten allgemeinen Formel kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen −25 und 250°C, vorzugsweise jedoch bei Temperaturen zwischen −10°C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Erforderlichenfalls wird die anschließende Abspaltung eines Schutzrestes vorzugsweise hydrolytisch durchgeführt, zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen −10 und 120°C, z.B. bei Temperaturen zwischen Raumtemperatur und der Siedetemperatur des Reaktionsgemisches.

Ein als Schutzrest verwendeter tert.Butylrest kann auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure abgespalten werden.

Desweiteren kann ein als Schutzrest verwendeter Benzylrest auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid abgespalten werden.

Die anschließende Kristallisation erfolgt in situ aus dem Äthanol/Wasser-haltigem Reaktionsgemisch oder, wie auch eine abschließende Umkristallisation, durch Lösen in einem Äthanol/Wasser-Gemisch, gegebenenfalls unter Erwärmen, und Abkühlen samt gegebenenfalls Anreiben und/oder Animpfen (Form B), oder durch Lösen in Aceton, und Zugabe von Petroläther (form A) oder durch Lösen (gegebenenfalls unter Erwärmen) in Methanol, anschließendes Abkühlen samt Anreiben und/oder Animpfen sowie Erwärmen des isolierten festen Methanol-Adduktes, vorzugsweise im Vakuum, in Gegenwart eines Trocknungsmittels, wie z.B. Phosphorpentoxid (Form C).

Die als Ausgangsstoffe verwendeten Verbindungen erhält man nach literaturbekannten Verfahren.

Wie bereits eingangs erwähnt, weisen die neuen festen Formen der 2-Äthoxy-4-[N-(1-(2-piperidino-phenyl)-3-methyl-1-butyl)-aminocarbonylmethyl]-benzoesäure wertvolle pharmakologische Eigenschaften auf, nämlich eine Wirkung auf den Intermédiärstoffwechsel, insbesondere jedoch eine blutzuckersenkende Wirkung.

Beispielsweise wurde die hochschmelzende Form (B) der Verbindung

2-Äthoxy-4-[N-(1-(2-piperidino-phenyl)-3-methyl-1-butyl)-aminocarbonylmethyl]-benzoesäure

auf ihre blutzuckersenkende Eigenschaft wie folgt untersucht:

1. Blutzuckersenkende Wirkung

Die blutzuckersenkende Wirkung der zu untersuchenden Substanz wurde an weiblichen Ratten eigener Zucht mit dem Gewicht von 180—220 g geprüft, welche 24 Stunden vor Versuchsbeginn nüchtern gesetzt wurden. Die zu untersuchende Substanz wurde unmittelbar vor Versuchsbeginn in 1,5%iger Methylcellulose suspendiert und per Schlundsonde appliziert.

Die Blutentnahme erfolgte unmittelbar vor Substanzapplikation sowie 1, 2, 3 und 4 Stunden danach, jeweils aus dem retroorbitalen Venenplexus. Hiervon wurden jeweils 50 µl mit 0,5 ml 0,33 N Perchlorsäure enteiweißt und zentrifugiert. Im Überstand wurde Glukose nach der Hexokinase-Methode mit Hilfe eines Analysenphotometers bestimmt. Die statistische Auswertung erfolgte nach dem t-Test nach Student mit $p = 0{,}05$ als Signifikanzgrenze.

Die nachfolgende Tabelle enthälkt die gefundenen Werte in Prozent gegenüber Kontrolle:

| | 0,1 mg/kg | | | |
|---|---|---|---|---|
| Substanz | 1 | 2 | 3 | 4 h |
| B | -38 | -44 | -41 | -40 |

2. Akute Toxizität

Bei weiblichen und männlichen Mäusen eigener Zucht mit dem Gewicht von 20—26 g wurde die toxische Wirkung nach oraler Gabe (Suspension in 1%iger Methylcellulose) einer einmaligen Dosis bei einer Nachbeobachtungszeit von 14 Tagen geprüft:

| Substanz | orientierende akute Toxizität |
|---|---|
| B | > 1000 mg/kg p.o. (0 von 6 Tieren gestorben) |

Aufgrund ihrer pharmakologischen Eigenschaften eignen sich die neuen festen Formen der 2-Äthoxy-4-[N-(1-(2-piperidinophenyl)-3-methyl-1-butyl)-aminocarbonylmethyl]-benzoesäure erfindungsgemäß zur Behandlung des Diabetes mellitus. Hierzu lassen sie sich, gegebenenfalls in Kombination mit anderen Wirksubstanzen, in die üblichen galenischen Zubereitungsformen wie Tabletten, Dragées, Kapseln, PUlver oder Suspensionen einarbeiten. Die Einzeldosis am Erwachsenen beträgt hierbei 1—50 mg, vorzugsweise jedoch 2,5—20 mg, 1 oder 2 mal täglich.

Die nachfolgenden Beispiele sollen die Erfindung näher erläutern:

Vorbemerkung:

Die Schmelzpunkte wurden in einem Electrothermal®-Schmelzpunktapparat unter visueller Betrachtung der in einem einseitig zugeschmolzenen Kappilarröhrchen befindlichen Substanzprobe bestimmt.

Beispiel 1

2-Äthoxy-4-[N-(1-(2-piperidino-phenyl)-3-methyl-1-butyl)-aminocarbonylmethyl]-benzoesäure-äthylester

Zu einer Lösung von 2,9 g (11,9 mMol) 3-Methyl-1-(2-piperidino-phenyl)-1-butylamin in 29 ml Acetonitril gibt man nacheinander 3 g (11,9 mMol) 3-Äthoxy-4-äthoxycarbonyl-phenylessigsäure, 3,7 g (14,3 mMol) Triphenylphosphin, 3,3 ml (23,8 mMol) Triäthylamin und 1,15 ml (11,9 mMol) Tetrachlorkohlenstoff. Anschließend rührt man 15 Stunden bei Raumtemperatur, entfernt dann im Vakuum das Lösungsmittel und verteilt den Rückstand zwischen Äthylacetat und Wasser. Den organischen Extrakt trocknet man über Natriumsulfat, filtriert und dampft im Vakuum ein. Den Eindampfrückstand reinigt man durch Säulenchromatographie an Kieselgel (Toluol/Aceton = 10/1).

Ausbeute: 4,9 g (85% der Theorie),
Schmelzpunkt: 143—145°C (Petroläther)

| | | | |
|---|---|---|---|
| Ber.: | C 72,47 | H 8,39 | N 5,83 |
| Gef.: | 72,37 | 8,45 | 6,07 |

Beispiel 2

Hochschmelzende Form (B) von 2-Äthoxy-4-[N-(1-(2-piperidinophenyl)-3-methyl-1-butyl)-aminocarbonylmethyl]-benzoesäure

Man rührt das Gemisch aus 4,7 g (9,7 mMol) 2-Äthoxy-4-[N-(1-(2-piperidino-phenyl)-3-methyl-1-butyl)-aminocarbonylmethyl]benzoesäure-äthylester und 14,7 ml 1N-Natronlauge in 47 ml Äthanol 2 Stunden bei 60°C, neutralisiert dann mit 14,7 ml 1N-Salzsäure und kühlt auf 0°C ab. Man filtriert vom ausgefallenen

farblosen Kirstillisat ab, wäscht mit Eiswasser und mit wenig eiskaltem Äthanol und trocknet bei 100°C/1 Torr (1,33 mbar).

Ausbeute: 3,9 g (88% der Theorie),
Schmelzpunkt: 140—142°C

| | | | |
|---|---|---|---|
| Ber. | C 71,65 | H 8,02 | N 6,19 |
| Gef. | 71,90 | 8,08 | 6,34 |

Bei weiterer Umkristallisation aus Äthanol/Wasser (2/1) bleibt der Schmelzpunkt konstant.

Beispiel 3

Niedrigschmelzende Form (A) von 2-Äthoxy-4-[N-(1-(2-piperidino-phenyl)-3-methyl-1-butyl)-aminocarbonylmethyl]-benzoesäure

Man löst 1,0 g der hochschmelzenden Form (B) von 2-Äthoxy-4-[N-(1-(2-piperidino-phenyl)-3-methyl-1-butyl)-aminocarbonylmethyl]-benzoesäure bei Raumtemperatur in 5 ml Aceton und fügt 5 ml Petroläther (60—70°C) zu. Beim Anreiben tritt allmählich Kristallisation ein. Man fügt nochmals die gleiche Menge Petroläther zu und filtriert nach beendeter Kristallisation ab. Man wäscht mit Petroläther und trocknet das fast farblose Kristallisat 2 Stunden bei 60°C/0,1 Torr (0,13 mbar).

Ausbeute: 0,7 g,
Schmelzpunkt: 95—98°C (klar ab 135°C)

| | | | |
|---|---|---|---|
| Ber. | C 71,65 | H 8,02 | N 6,19 |
| Gef. | 71,80 | 8,04 | 5,92 |

Die Spektren dieser Form (siehe Abbildungen A und A') sind identisch mit den Spektren der in der eingangs erwähnten nicht vorveröffentlichten Europäischen Offenlegungsschrift beschriebenen Form (A) vom Schmelzpunkt 90—92°C.

Beispiel 4

Hochschmelzende Form (B) von 2-Äthoxy-4-[N-(2-(2-piperidinophenyl)-3-methyl-1-butyl)-aminocarbonylmethyl]-benzoesäure

Man löst 1,0 g der niedrigschmelzenden Form (A) von 2-Äthoxy-4-[N-(1-(2-piperidino-phenyl)-3-methyl-1-butyl)-aminocarbonylmethyl]-benzoesäure unter Erwärmen auf dem Dampfbad in 10 ml Äthanol/Wasser (2/1). Dann kühlt man auf 0°C ab, wobei Kristallisation eintritt. Man filtriert, wäscht mit wenig eiskaltem Äthanol und trocknet bei 100°C/1 Torr (1,33 mbar).

Ausbeute: 0,8 g,
Schmelzpunkt: 140—142°C

Beispiel 5

Schaumartige Form (C) von 2-Äthoxy-4-[N-(1-(2-piperidinophenyl)-3-methyl-1-butyl)-aminocarbonylmethyl]-benzoesäure

Man löst 1,5 g der hochschmelzenden Form (B) von 2-Äthoxy-4-[N-(1-(2-piperidino-phenyl)-3-methyl-1-butyl)-aminocarbonylmethyl]-benzoesäure unter Erwärmen in 5 ml Methanol. Dann kühlt man unter Anreiben auf 0°C ab. Vom ausgefallenen Kristallisat filtriert man ab, wäscht mit wenig kaltem Methanol und trocknet 2 Stunden bei 60°C/0,1 Torr (0,13 mbar).

Ausbeute an Addukt (mit 1 × $CH_3OH$): 1,2 g,
Schmelzpunkt: 85—90°C

| | | | |
|---|---|---|---|
| Ber. (× 1 $CH_3OH$): | C 69,39 | H 8,32 | N 5,78 |
| Gef. | 69,20 | 8,20 | 5,92 |

Das vorstehende Addukt wird durch 24-stündiges Erwärmen bei 60°C/5 Torr (6,7 mbar) über Phosphorpentoxid in die methanol-freie schaumartige Form (C) übergeführt.

Schmelzbereich: 75—85°C

| | | | |
|---|---|---|---|
| Ber. | C 71,65 | H 8,02 | N 6,19 |
| Gef. | 71,82 | 8,06 | 6,03 |

Beispiel 6

Tabletten mit 5 mg 2-Äthoxy-4-[N-(1-(2-piperidino-phenyl)-3-methyl-1-butyl)-aminocarbonylmethyl]-benzoesäure mit dem Schmelzpunkt von 140—142°C oder von 75—85°C

Zusammensetzung:
1 Tablette enthalt:

| | | |
|---|---|---|
| Wirksubstanz | (1) | 5,0 mg |
| Maisstärke | (2) | 62,0 mg |
| Milchzucker | (3) | 48,0 mg |
| Polyvinylpyrrolidon | (4) | 4,0 mg |
| Magnesiumstearat | (5) | 1,0 mg |
| | | 120,0 mg |

Herstellungsverfahren:

1, 2, 3 und 4 werden gemischt und mit Wasser befeuchtet. Die feuchte Mischung wird durch ein Sieb mit 1,5 mm Maschenweite gedrückt und bei ca. 45°C getrocknet. Das trockene Granulat wird durch ein Sieb mit 1,0 mm Maschenweite geschlagen und mit 5 vermischt. Die fertige Mischung preßt man auf einer Tablettenpresse mit Stempeln von 7 mm Durchmesser, die mit einer Teilkerbe versehen sind, zu Tabletten.

Tablettengewicht: 120 mg

Beispiel 7

Dragées mit 2,5 mg 2-Äthoxy-4-[N-(1-(2-piperidino-phenyl)-3-methyl-1-butyl)-aminocarbonylmethyl]-benzoesäure mit dem Schmelzpunkt von 140—142°C oder von 75—85°C

1 Drageékern enthält:

| | | |
|---|---|---|
| Wirksubstanz | (1) | 2,5 mg |
| Kartoffelstärke | (2) | 44,0 mg |
| Milchzucker | (3) | 30,0 mg |
| Polyvinylpyrrolidon | (4) | 3,0 mg |
| Magnesiumstearat | (5) | 0,5 mg |
| | | 80,0 mg |

Herstellungsverfahren:

1, 2, 3 und 4 werden gut gemischt und mit Wasser befeuchtet. Die feuchte Masse drückt man durch ein Sieb mit 1 mm Maschenweite, trocknet bei ca. 45°C und schlägt das Granulat anschließend durch dasselbe Sieb. Nach dem Zumischen von 5 werden auf einer Tablettiermaschine gewölbte Drageékerne mit einem Durchmesser von 6 mm gepreßt. Die so hergestellten Drageékerne werden auf bekannte Weise mit einer Schicht überzogen, die im wesentlichen aus Zucker und Talkum besteht. Die fertigen Dragées werden mit Wachs poliert.

Drageégewicht: 120 mg

Beispiel 8

Tabletten mit 10 mg 2-Äthoxy-4-[N-(1-(2-piperidino-phenyl)-3-methyl-1-butyl)-aminocarbonylmethyl]-benzoesäure mit dem Schmelzpunkt von 140—142°C oder von 75—85°C

Zusammensetzung:
1 Tablette enthält:

| | |
|---|---|
| Wirksubstanz | 10,0 mg |
| Milchzucker pulv. | 70,0 mg |
| Maisstärke | 31,0 mg |
| Polyvinylpyrrolidon | 8,0 mg |
| Magnesiumstearat | 1,0 mg |
| | 120,0 mg |

Herstellungsverfahren:

Die Mischung aus der Wirksubstanz, Milchzucker und Maisstärke wird mit einer 20 %igen Lösung von Polyvinylpyrrolidon in Wasser befeuchtet. Die feuchte Masse wird durch ein Sieb mit 1,5 mm Maschenweite granuliert und bei 45°C getrocknet. Das getrocknete Granulat wird durch ein Sieb mit 1 mm Maschenweite gerieben und mit Magnesiumstearat homogen vermischt.

Tablettengewicht: 120 mg
Stempel: 7 mm Durchmesser mit Teilkerbe

Beispiel 9

Dragées mit 5 mg 2-Äthoxy-4-[N-(1-(2-piperidino-phenyl)-3-methyl-1-butyl)-aminocarbonylmethyl]-benzoesäure mit dem Schmelzpunkt von 140—142°C oder von 75—85°C

1 Dragéekern enthält:

| | |
|---|---|
| Wirksubstanz | 5,0 mg |
| Calciumphosphat sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren:

Die Mischung aus der Wirksubstanz, Calciumphosphat und Maisstärke wird mit einer 15 %igen Lösung von Polyvinylpyrrolidon in Wasser befeuchtet. Die feuchte Masse wird durch ein Sieb mit 1 mm Maschenweite geschlagen, bei 45°C getrocknet und anschließend durch dasselbe Sieb gerieben. Nach dem Vermischen mit der angegebenen Menge Magnesiumstearat werden daraus Dragéekerne gepreßt.

Kerngewicht: 130 mg
Stempel: 7 mm Durchmesser
Auf die so hergestellten Dragéekerne wird auf bekannte Art eine Schicht aus Zucker und Talkum aufgetragen. Die fertigen Dragées werden mit Wachs poliert.
Dragéegewicht: 180 mg

**Patentansprüche**

1. Zwei neue feste Formen von 2-Äthoxy-4-[N-(1-(2-piperidino-phenyl)-3-methyl-1-butyl)-aminocarbonylmethyl]-benzoesäure, wobei die als Form (B) bezeichnete Form durch einen Schmelzpunkt von 140—142°C sowie durch das IR(KBr)-Spektrum gemäß Abbildung B′ und die als Form (C) durch einen Schmelzpunkt von 75—85°C sowie durch das IR(KBr)-Spektrum gemäß Abbildung C′ gekennzeichnet ist.

2. Form (B) von 2-Äthoxy-4-[N-(1-(2-piperidino-phenyl)-3-methyl-1-butyl)-aminocarbonylmethyl]-benzoesäure gemäß Anspruch 1 mit einem Schmelzpunkt von 140—142°C.

3. Form (C) von 2-Äthoxy-4-[N-(1-(2-piperidino-phenyl)-3-methyl-1-butyl)-aminocarbonylmethyl]-benzoesäure gemäß Anspruch 1 mit einem Schmelzpunkt von 75—85°C.

4. Arzneimittel, enthaltend eine Verbindung gemäß den Ansprüchen 1 bis 3 neben gegebenenfalls einem oder mehreren inerten Trägerstoffen und/oder Verdünnungsmitteln.

5. Verwendung einer Verbindung gemäß den Ansprüchen 1 bis 3 zur Herstellung eines Arzneimittels, welches zur Behandlung des Diabetes mellitus geeignet ist.

6. Verfahren zur Herstellung eines Arzneimittels gemäß Anspruch 4, dadurch gekennzeichnet, daß eine Verbindung gemäß den Ansprüchen 1 bis 3 in einen oder mehrere inerte Trägerstoffe und/oder Verdünnungsmittel eingearbeitet wird.

7. Verfahren zur Herstellung der Verbindung gemäß Anspruch 2, dadurch gekennzeichnet, daß 2-Äthoxy-4-[N-(1-(2-piperidino-phenyl)-3-methyl-1-butyl)-aminocarbonylmethyl]benzoesäure durch Lösen in Äthanol/Wasser (2/1) kristallisiert wird.

8. Verfahren zur Herstellung der Verbindung gemäß Anspruch 3, dadurch gekennzeichnet, daß 2-Äthoxy-4-[N-(1-(2-piperidino-phenyl)-3-methyl-1-butyl)-aminocarbonylmethyl]benzoesäure durch Lösen in Methanol kristallisiert und anschließend das erhaltene Kristallisat bei 60°C im Vakuum über Phosphorpentoxid getrocknet wird.

9. Verfahren gemäß den Ansprüchen 7 und 8, dadurch gekennzeichnet, daß 2-Äthoxy-4-[N-(1-(2-piperidino-phenyl)-3-methyl-1-butyl)-aminocarbonyl-methyl]-benzoesäure durch Umsetzung von 3-Methyl-1-(2-piperidino-phenyl)-1-butylamin mit einer Carbonsäure der allgemeinen Formel

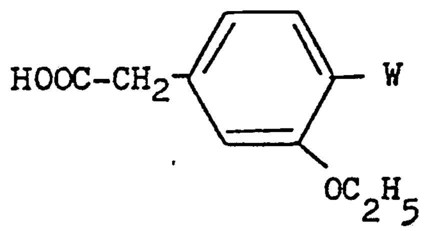

in der

W eine Carboxygruppe oder eine durch einen Schutzrest geschützte Carboxygruppe darstellt, oder mit deren gegebenenfalls im Reaktionsgemisch hergestellten reaktionsfähigen Derivaten hergestellt und erforderlichenfalls ein verwendeter Schutzrest abgespalten wird.

10. Verfahren gemäß Anspruch 9, dadurch gekennzeichnet, daß die Umsetzung in einem Lösungsmittel, in Gegenwart eines die Säure aktivierenden oder eines wasserentziehenden Mittels gegebenenfalls in Gegenwart einer anorganischen oder tertiären organischen Base und bei Temperaturen zwischen −25 und 250°C, vorzugsweise bei Temperaturen zwischen −10°C und der Siedetemperatur des verwendeten Lösungsmittels, durchgeführt wird.

**Revendications**

1. Deux nouvelles formes solides de l'acide 2-éthoxy-4-[N-1-(2-pipéridino-phényl)-3-méthyl-1-butyl)-aminocarbonylméthyl]-benzoïque, la forme désignée en tant que forme (B) étant caractérisée par un point de fusion de 140—142°C ainsi que par le spectre IR (KBr) selon la figure B' et la forme désignée en tant que forme (C) étant caractérisée par un point de fusion de 75—85°C ainsi que par le spectre IR (KBr) selon la figure C'.

2. Forme (B) de l'acide 2-éthoxy-4-[N-(1-(2-pipéridino-phényl)-3-méthyl-1-butyl)-aminocarbonyl-méthyl]-benzoïque selon la revendication 1, présentant un point de fusion de 140—142°C.

3. Forme (C) de l'acide 2-éthoxy-4-[N-(1-(2-pipéridino-phényl)-3-méthyl-1-butyl)-aminocarbonyl-méthyl]-benzoïque selon la revendication 1, présentant un point de fusion de 75—85°C.

4. Médicament contenant un composé selon les revendications 1 à 3 conjointement, le cas échéant, à un ou plusieurs excipients et/ou diluants inertes.

5. Utilisation d'un composé selon les revendications 1 à 3 pour la fabrication d'un médicament qui convient au traitement du diabète mellitus.

6. Procédé pour la fabrication d'un médicament selon la revendication 4, caractérisé en ce qu'on incorpore un composé selon les revendications 1 à 3 dans un ou plusieurs excipients et/ou diluants inertes.

7. Procédé pour la préparation du composés selon la revendication 2, caractérisé en ce que l'acide 2-éthoxy-4-[N-(1-(2-pipéridino-phényl)-3-méthyl-1-butyl)-aminocarbonylméthyl]-benzoïque est cristallisé par dissolution dans de l'éthanol/eau (2/1).

8. Procédé pour la préparation du composé selon la revendication 3, caractérisé en ce que l'acide 2-éthoxy-4-[N-(1-(2-pipéridino-phényl)-3-méthyl-1-butyl)-aminocarbonylméthyl]-benzoïque est cristallisé par dissolution dans du méthanol puis les cristaux obtenus sont séchés à 60°C sous vide, sur pentoxyde de phosphore.

9. Procédé selon les revendications 7 et 8, caractérisé en ce que l'acide 2-éthoxy-4-[N-(1-(2-pipéridino-

phényl)-3-méthyl-1-butyl)-aminocarbonylméthyl]-benzoïque est préparé par réaction de la 3-méthyl-1-(2-pipéridino-phényl)-1-butylamine avec un acide carboxylique de formule générale

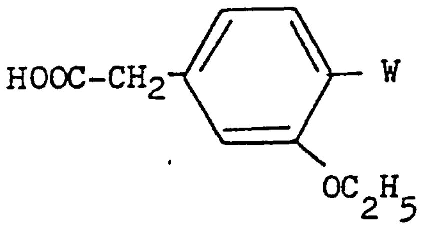

dans laquelle

W représente un groupe carboxy ou un groupe carboxy protégé par un radical protecteur, ou avec ses dérivés aptes à réagir, éventuellement préparés dans le mélange réactionnel et si nécessaire un groupe protecteur utilisé est clivé.

10. Procédé selon la revendication 9, caractérisé en ce que la réaction est effectuée dans un solvant, en présence d'un agent activant les acides ou d'un agent d'élimination de l'eau, éventuellement en présence d'une base non organique ou organique tertiaire et à des températures entre −25 et 250°C, de préférence à des températures entre −10°C et la température d'ébullition du solvant utilisé.

**Claims**

1. Two new solid forms of 2-ethoxy-4-[N-(1-(2-piperidino-phenyl)-3-methyl-1-butyl)-aminocarbonyl-methyl]-benzoic acid, the form designated (B) being characterised by the melting point of 140—142°C and by the IR(KBr) spectrum according to Figure B' and the form designated (C) being characterised by a melting point of 75—85°C and by the IR(KBr) spectrum according to Figure C'.

2. Form (B) of ethoxy-4-[N[(1-(2-piperidino-phenyl)-3-methyl-1-butyl)-aminocarbonylmethyl]-benzoic acid according to claim 1 with a melting point of 140—142°C.

3. Form (C) of 2-ethoxy-4-[N-((1-2-piperidino-phenyl)-3-methyl-1-butyl)-aminocarbonylmethyl]-benzoic acid according to claim 1 with a melting point of 75—85°C.

4. Pharmaceutical composition, containing a compound according to claims 1 to 3 optionally together with one or more inert carriers and/or diluents.

5. Use of a compound according to claims 1 to 3 for the preparation of the pharmaceutical composition suitable for the treatment of diabetes mellitus.

6. Process for preparing a pharmaceutical composition according to claim 4, characterised in that a compound according to claims 1 to 3 is incorporated in one or more inert carriers and/or diluents.

7. Process for preparing the compound according to claim 2, characterised in that 2-ethoxy-4-[N-(1-(2-piperidino-phenyl)-3-methyl-1-butyl)-aminocarbonylmethyl]benzoic acid is crystallised by dissolving in ethanol/water (2/1).

8. Process for preparing the compound according to claim 3, characterised in that 2-ethoxy-4-[N-(1-2-piperidino-phenyl)-3-methyl-1-butyl)-aminocarbonylmethyl]benzoic acid is crystallised by dissolving in methanol and subsequently the crystals obtained are dried at 60°C *in vacuo* over phosphorus pentoxide.

9. Process according to claims 7 and 8, characterised in that 2-ethoxy-4-[N-(1-(2-piperidino-phenyl)-3-methyl-1-butyl)-aminocarbonyl-methyl]benzoic acid is prepared by reacting 3-methyl-1-(2-piperidino-phenyl)-1-butylamine with a carboxylic acid of general formula

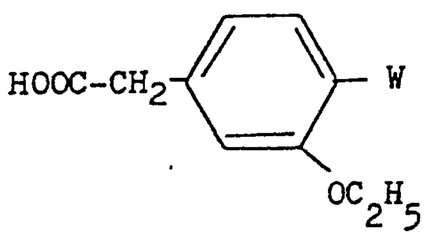

wherein

W represents a carboxy group or a carboxy group protected by a protecting group, or with reactive derivatives thereof optionally prepared in the reaction mixture and if necessary any protecting group used is split off.

10. Process according to claim 9, characterised in that the reaction is carried out in a solvent, in the presence of an acid-activating or dehydrating agent, optionally in the presence of an inorganic or tertiary organic base and at temperatures between −25 and 250°C, preferably at temperatures between −10°C and the boiling temperature of the solvent used.

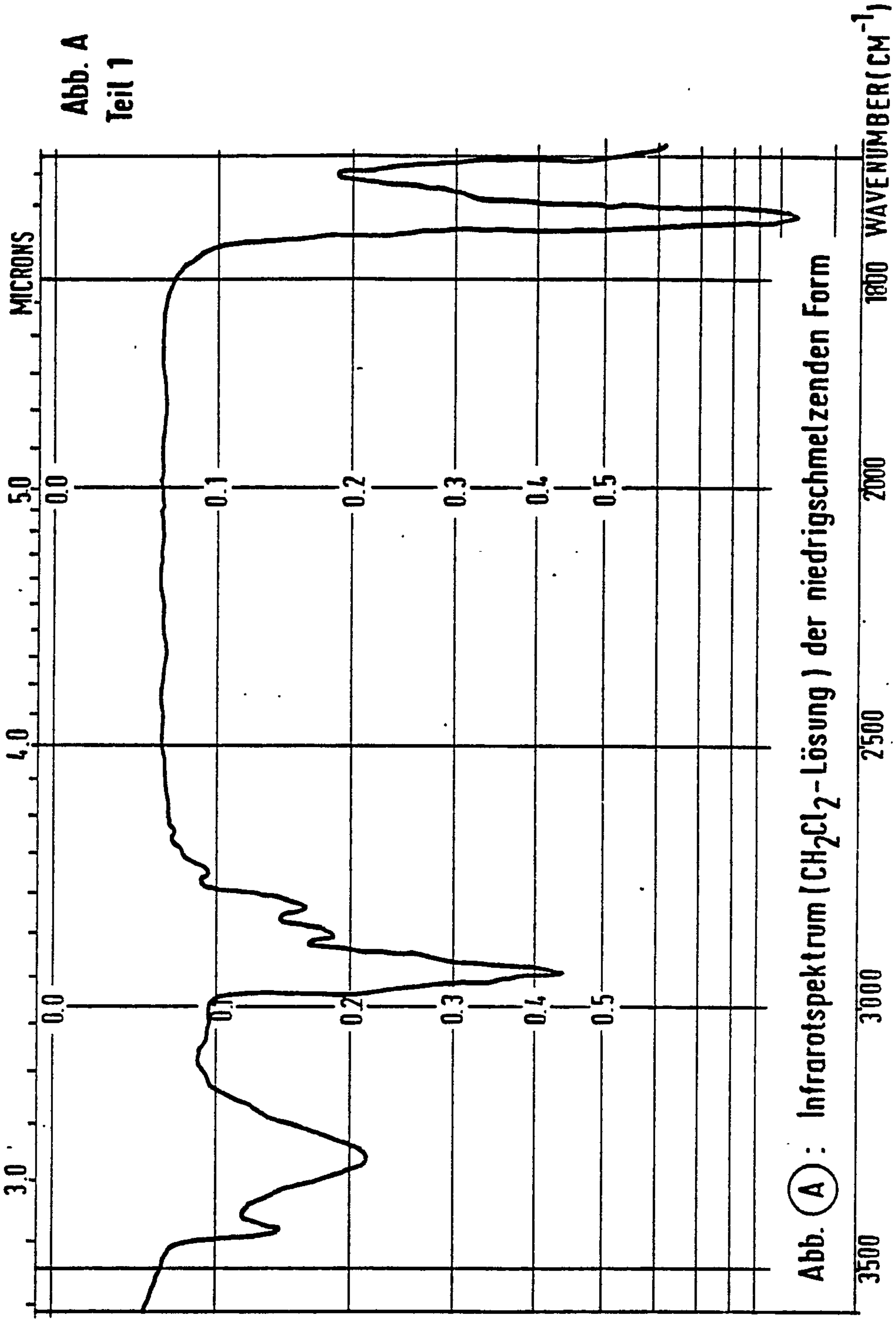


Abb. (A): Infrarotspektrum ($CH_2Cl_2$-Lösung) der niedrigschmelzenden Form

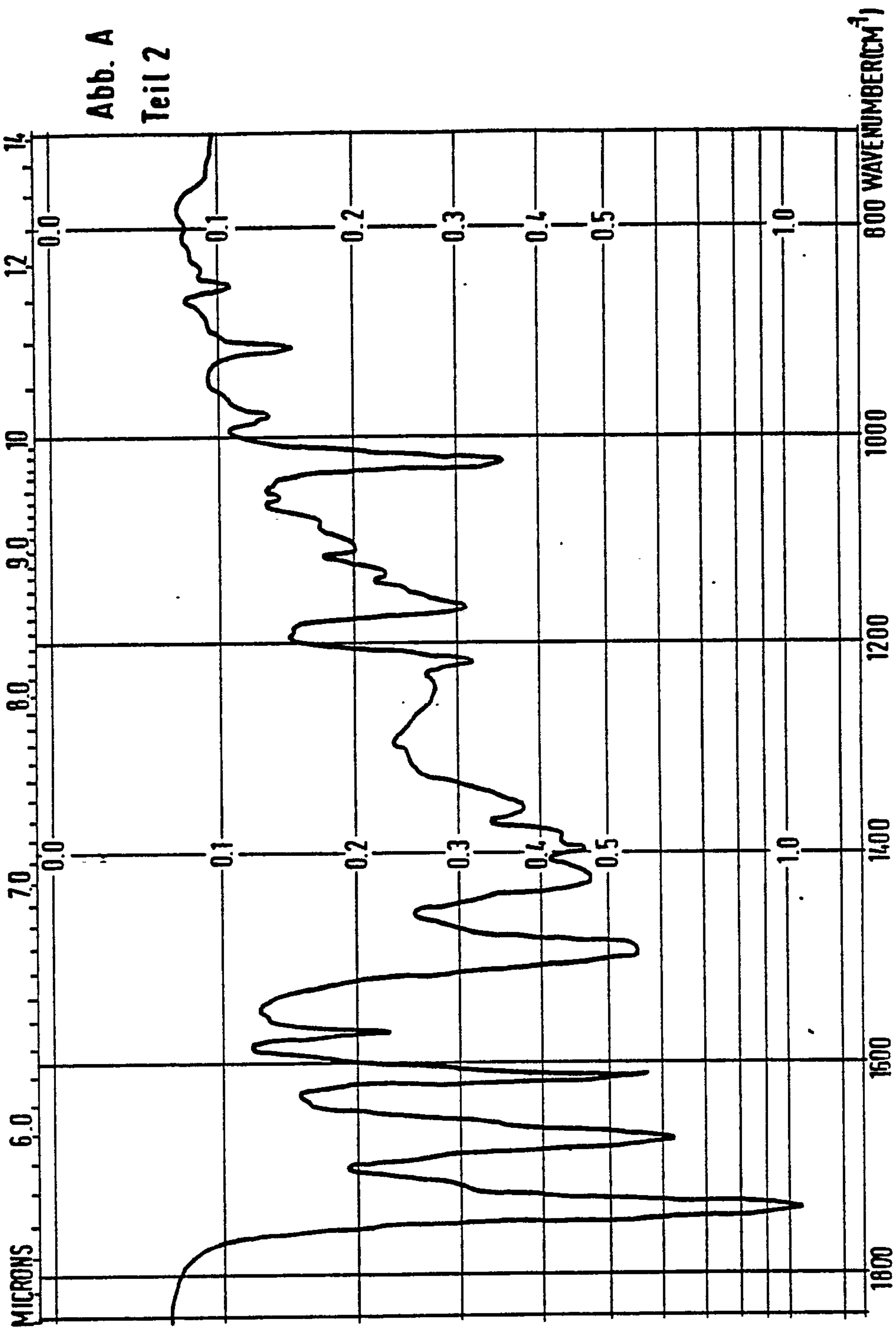
Abb. A
Teil 2
MICRONS
6.0
7.0
8.0
9.0
10
12
14
0.0
0.1
0.2
0.3
0.4
0.5
1.0
1800
1600
1400
1200
1000
800 WAVENUMBER(CM-1)

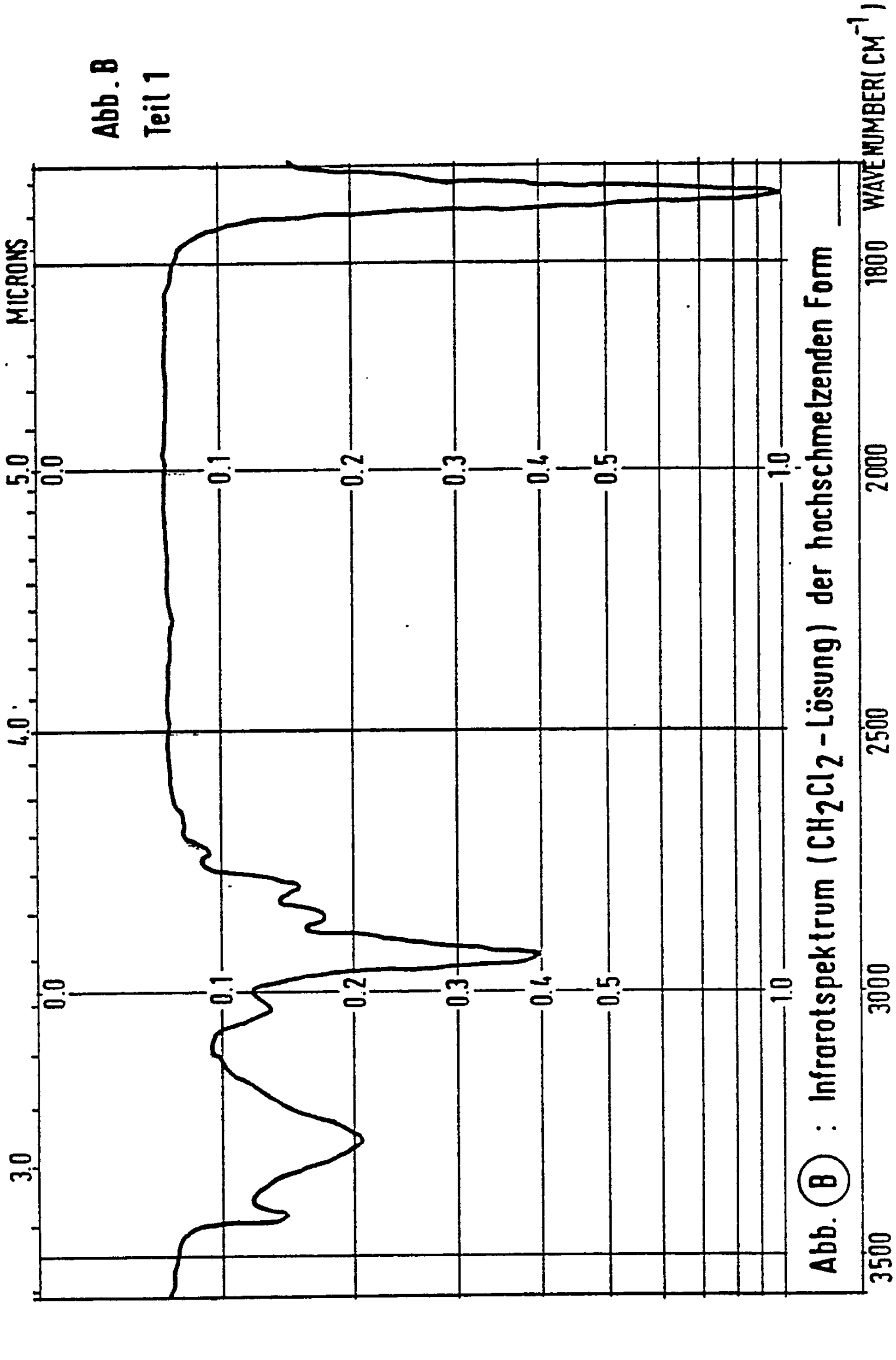


Abb. (B) : Infrarotspektrum ($CH_2Cl_2$-Lösung) der hochschmelzenden Form

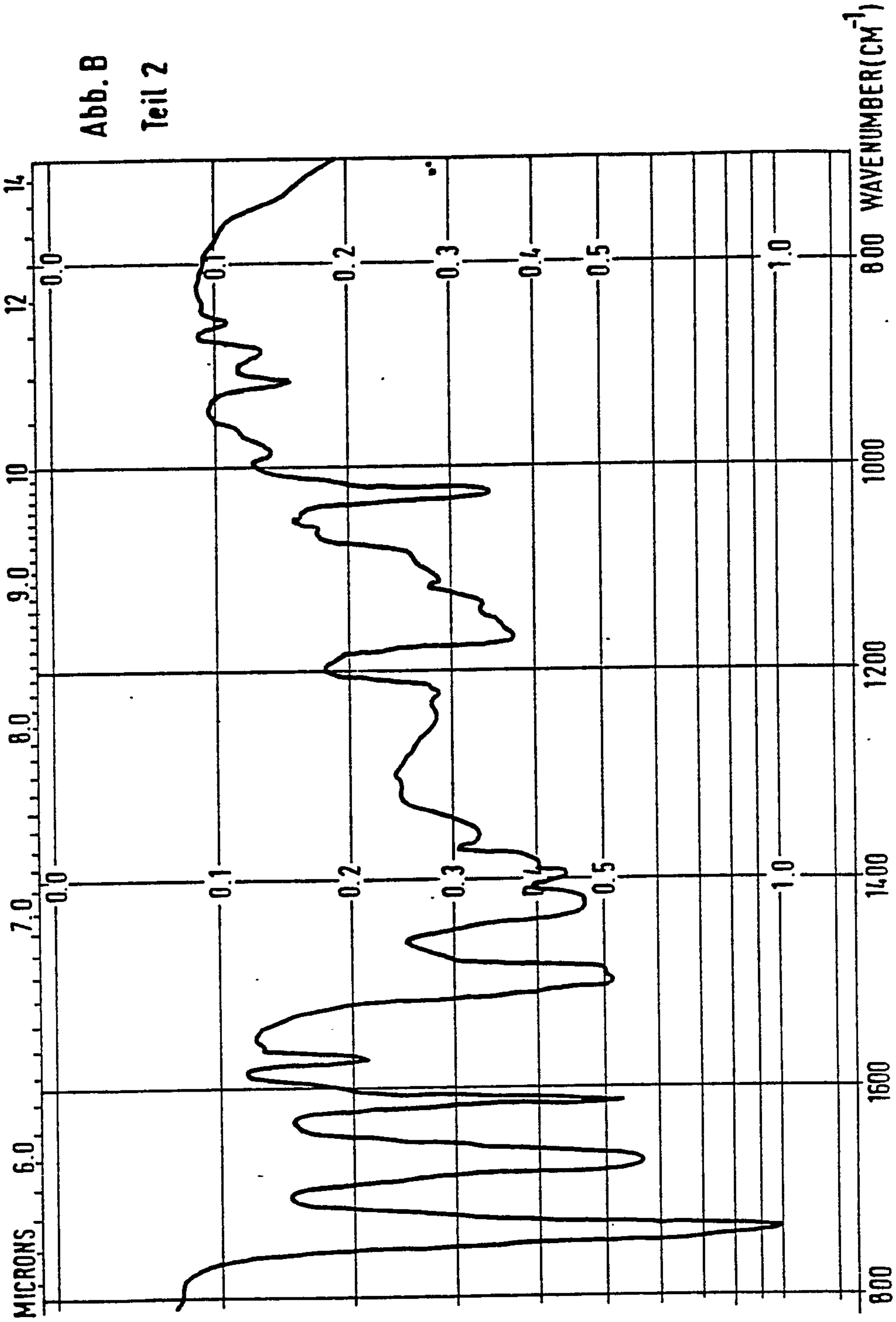
MICRONS
6.0
7.0
8.0
9.0
10
12
14
0.0
0.1
0.2
0.3
0.4
0.5
1.0
800
1600
1400
1200
1000
800 WAVENUMBER(CM$^{-1}$)

Abb. B
Teil 2

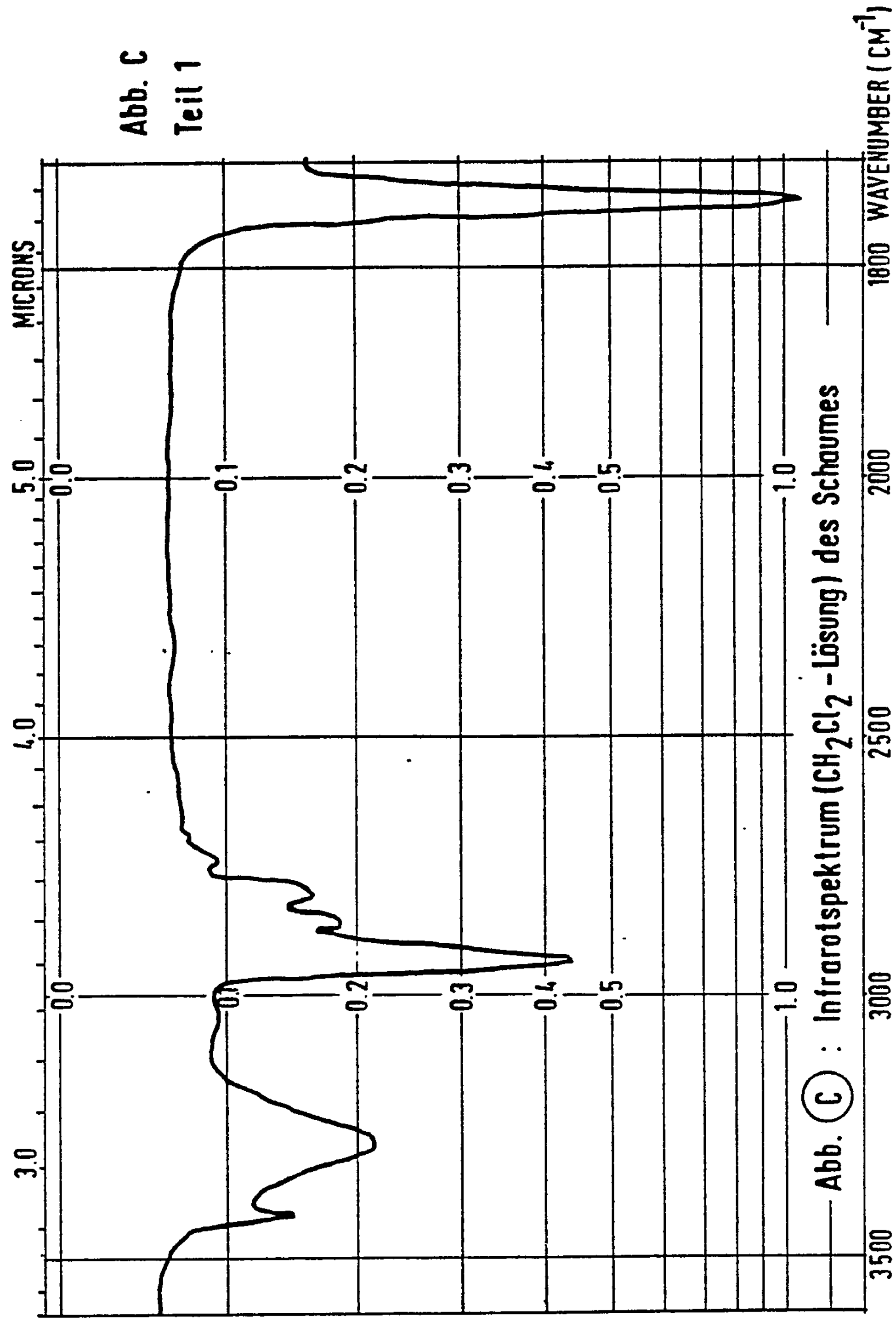


Abb. (C) : Infrarotspektrum ($CH_2Cl_2$-Lösung) des Schaumes

Abb. C
Teil 2

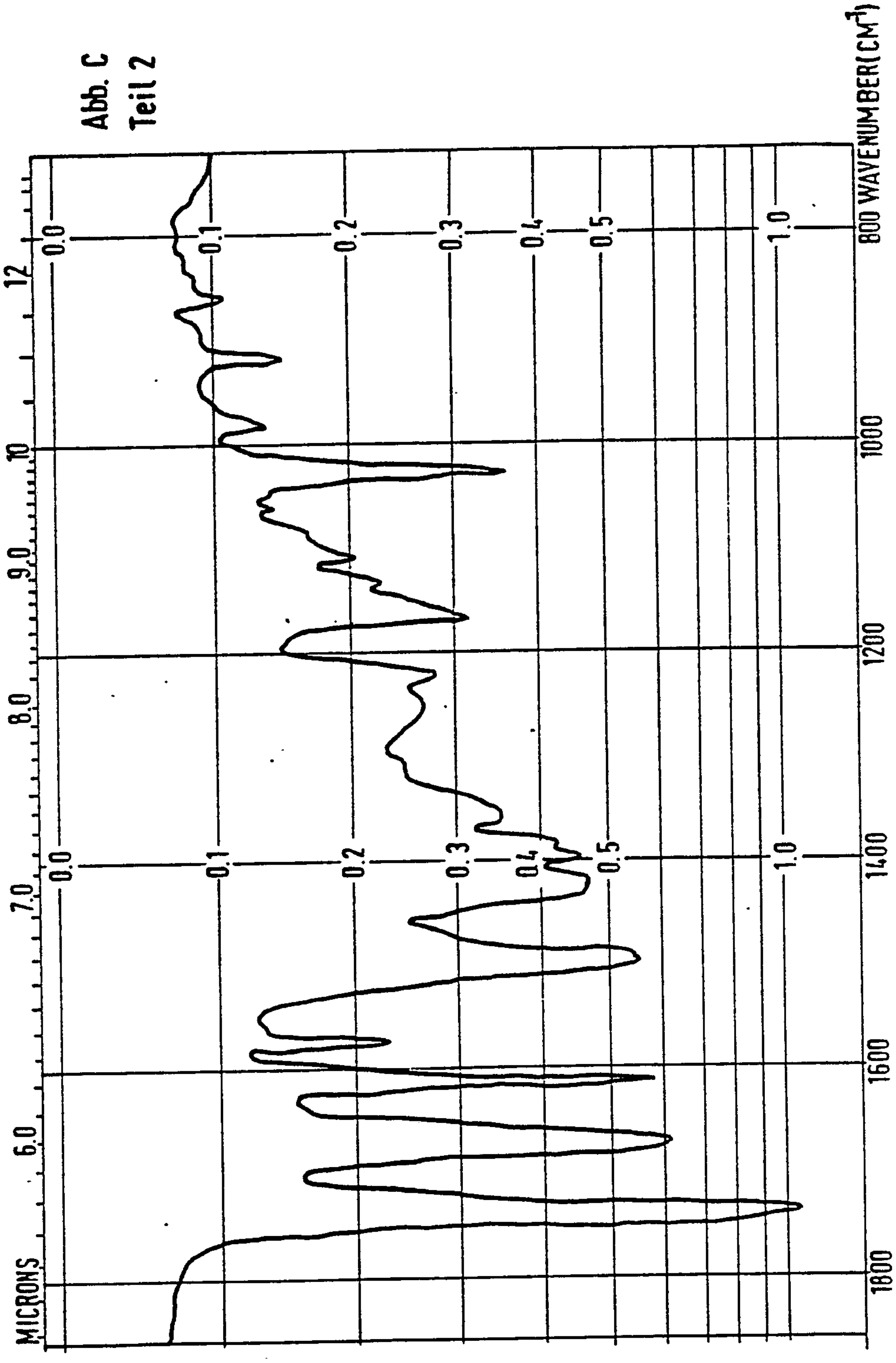
MICRONS
6.0
7.0
8.0
9.0
10
12
0.0
0.1
0.2
0.3
0.4
0.5
1.0
1800
1600
1400
1200
1000
800 WAVENUMBER(CM$^{-1}$)

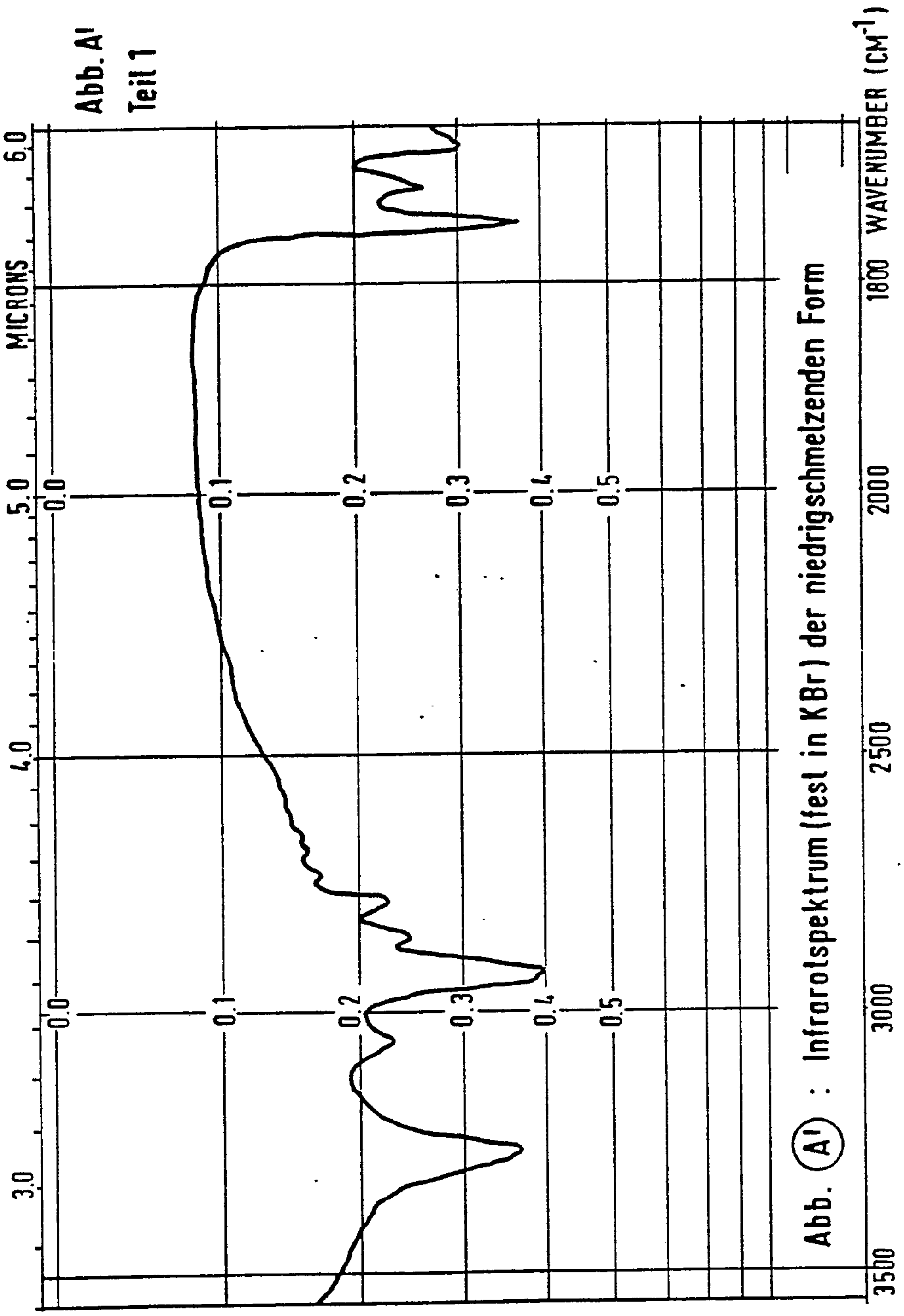


Abb. (A') : Infrarotspektrum (fest in KBr) der niedrigschmelzenden Form

Abb. A'
Teil 2

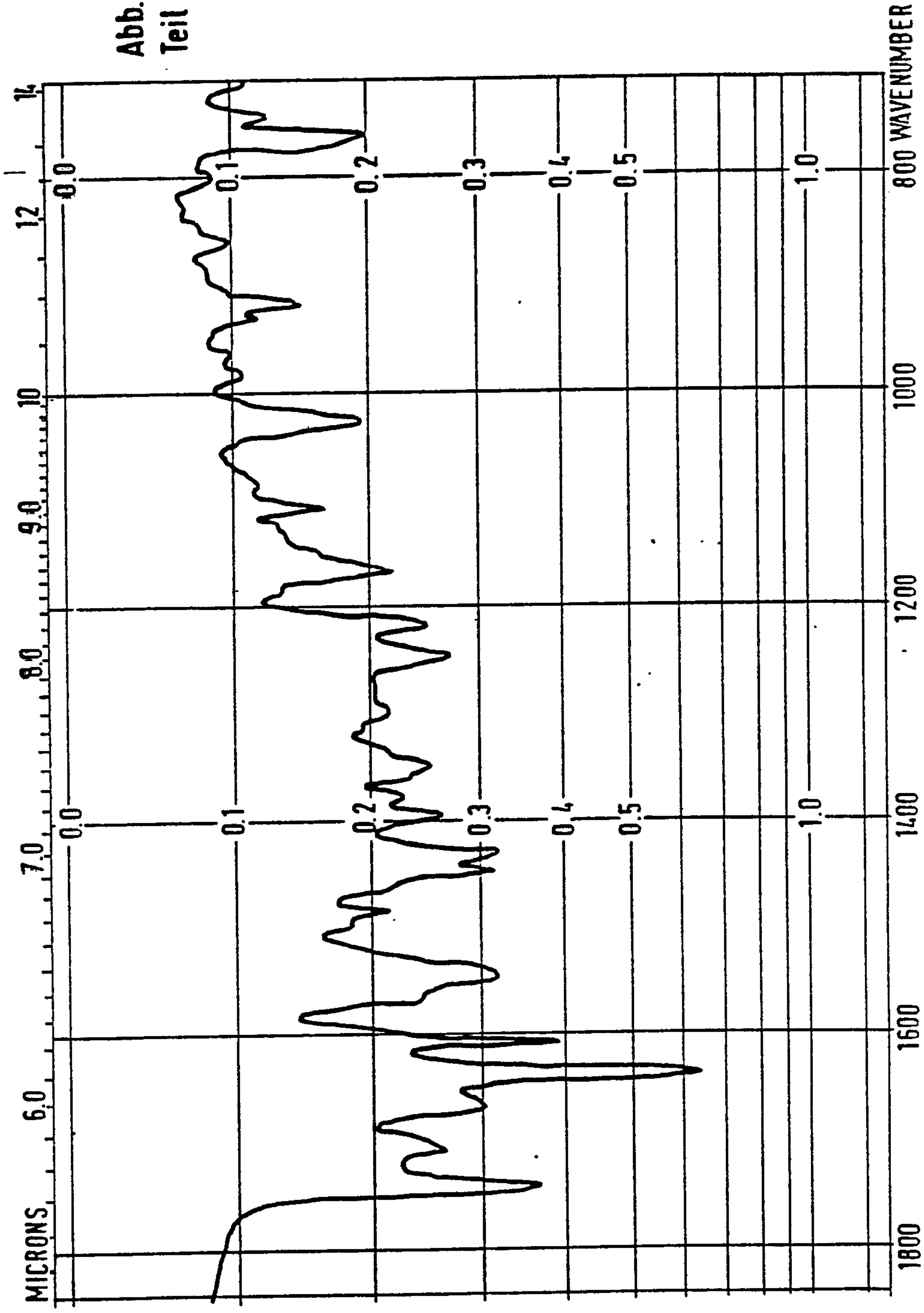
MICRONS
6.0
7.0
8.0
9.0
10
12
14
0.0
0.1
0.2
0.3
0.4
0.5
1.0
1800
1600
1400
1200
1000
800 WAVENUMBER (CM-1)

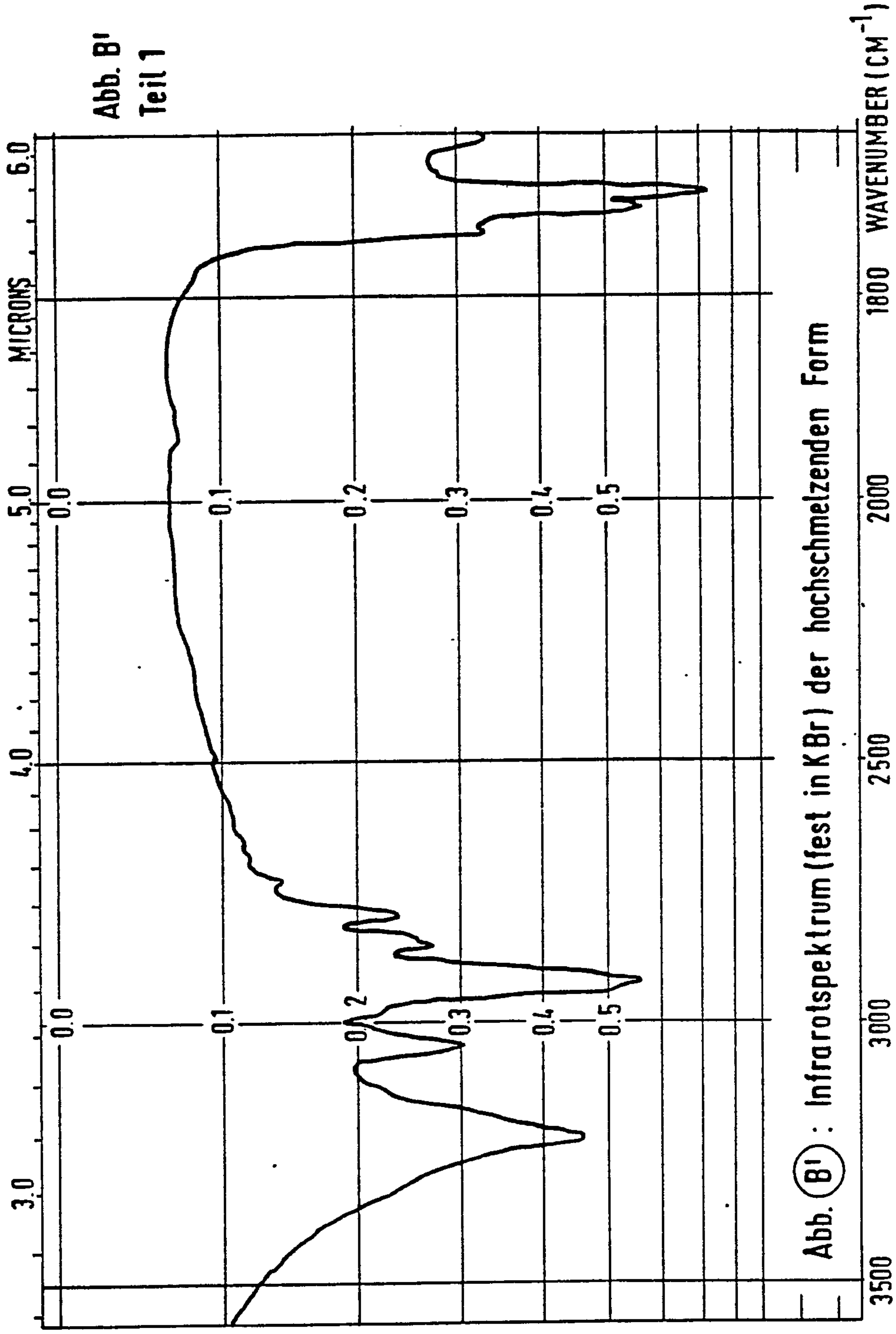


Abb. (B'): Infrarotspektrum (fest in KBr) der hochschmelzenden Form

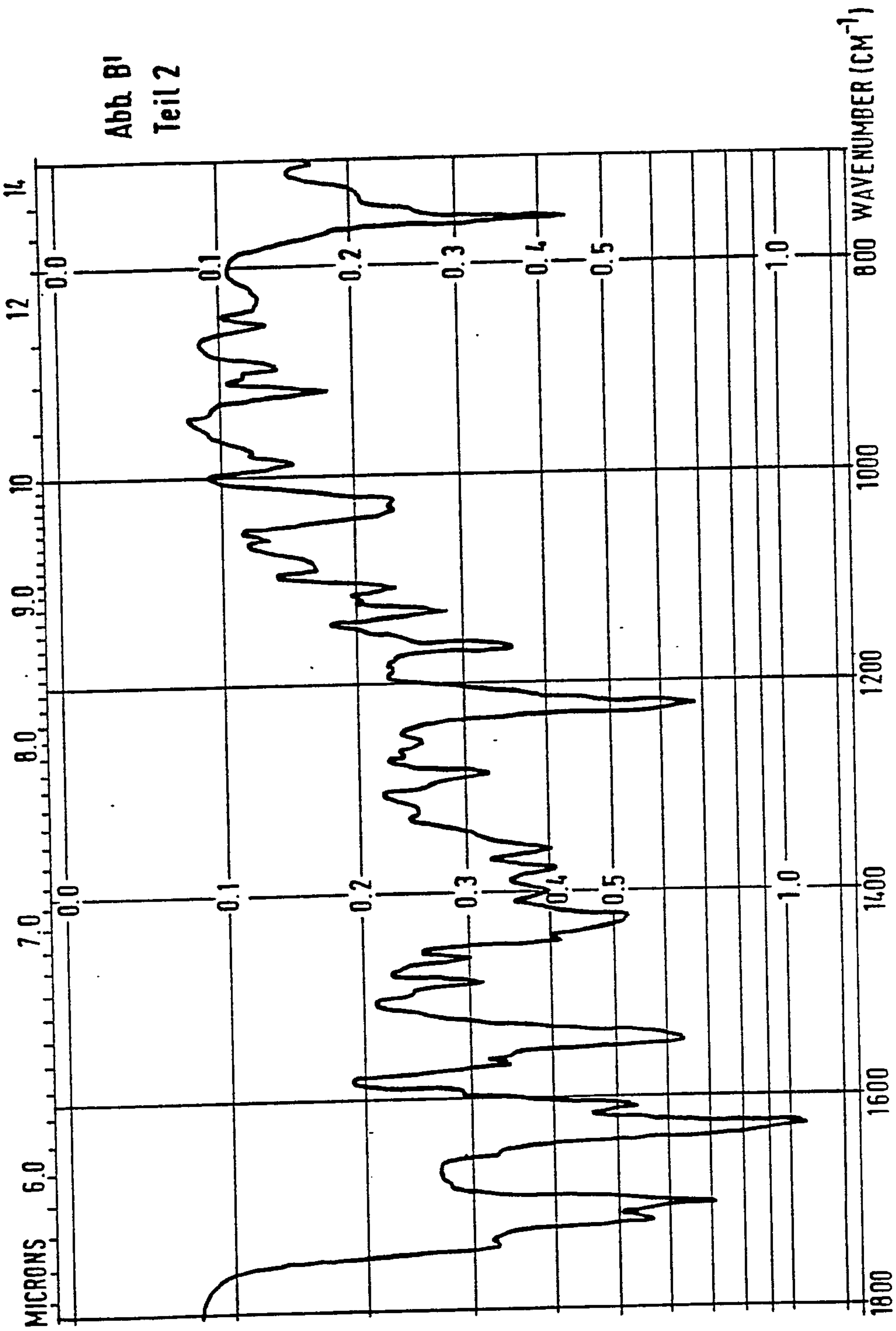
MICRONS
6.0
7.0
8.0
9.0
10
12
14
0.0
0.1
0.2
0.3
0.4
0.5
1.0
1800
1600
1400
1200
1000
800
WAVENUMBER (CM⁻¹)

Abb. B'
Teil 2

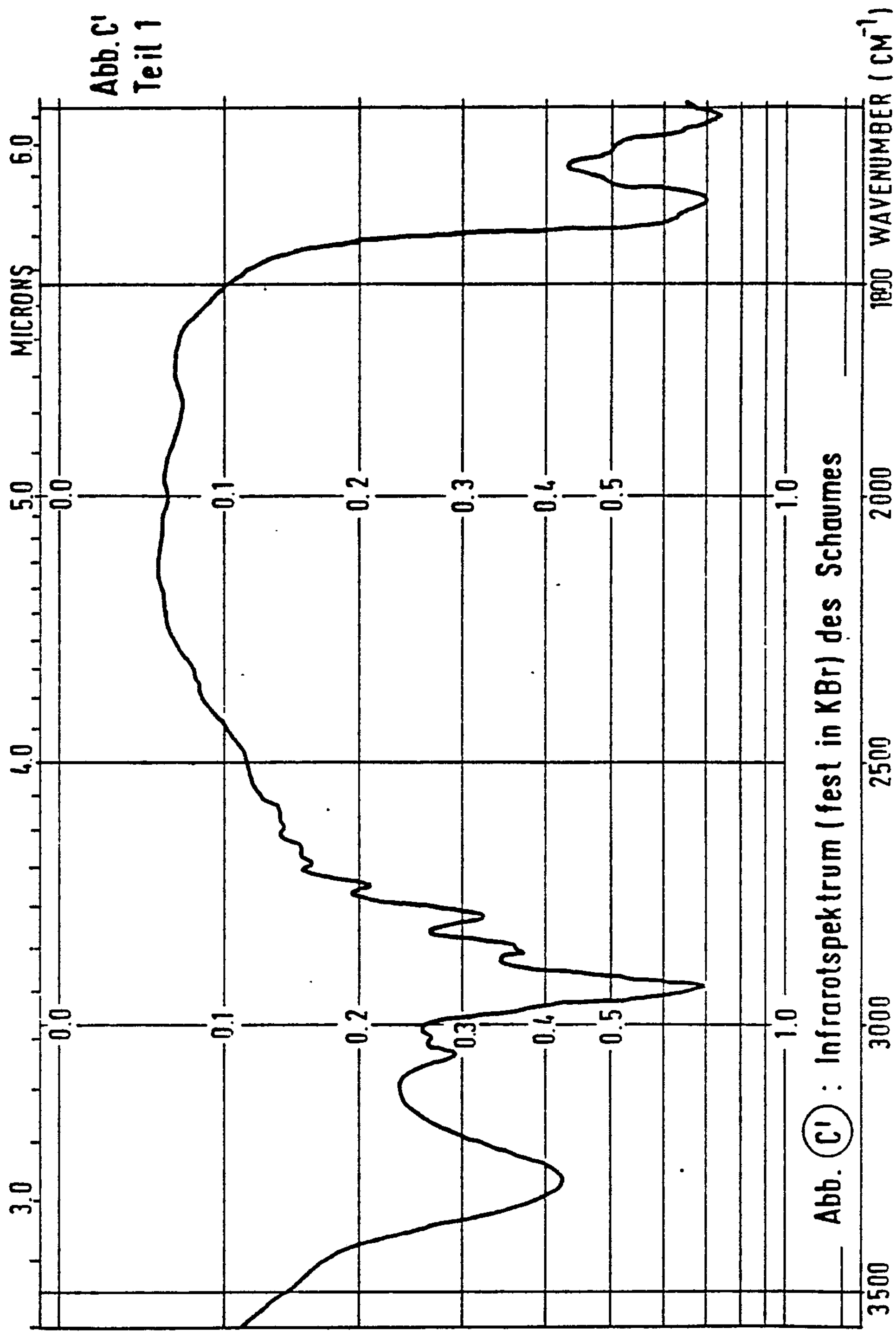


Abb. (C') : Infrarotspektrum (fest in KBr) des Schaumes

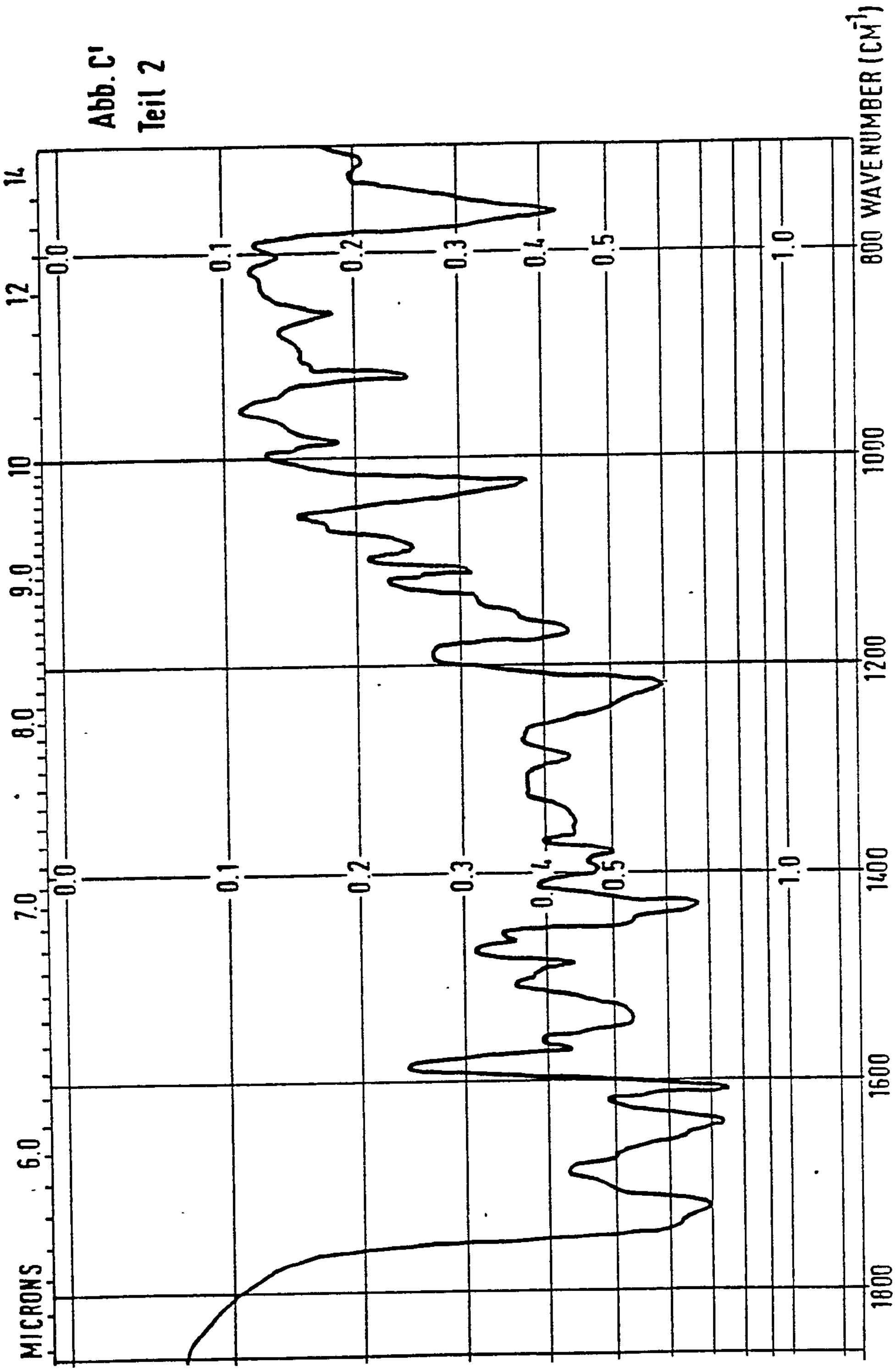
Abb. C'
Teil 2
MICRONS
6.0
7.0
8.0
9.0
10
12
14
0.0
0.1
0.2
0.3
0.4
0.5
1.0
1800
1600
1400
1200
1000
800 WAVENUMBER (CM-1)